# EUROPÄISCHE PATENTSCHRIFT

(11) **EP 2 043 989 B1**
(45) Veröffentlichungstag und Bekanntmachung des Hinweises auf die Patenterteilung: **03.11.2010**
(21) Anmeldenummer: 07787218.2
(22) Anmeldetag: 09.07.2007
(51) Int. Cl.: C07C 45/00, C07C 47/06, C07C 47/04, C07C 49/08

(54) **VERFAHREN ZUR DEHYDRIERUNG VON ALKOHOLEN**
A METHOD FOR DEHYDRATING ALCOHOLS
PROCÉDÉ POUR LA DÉSHYDRATATION D'ALCOOLS

(30) Priorität: 11.07.2006 EP 06116987
(43) Veröffentlichungstag der Anmeldung: 08.04.2009
(73) Patentinhaber: BASF SE, 67056 Ludwigshafen (DE)
(72) Erfinder: URTEL, Heiko, 68165 Mannheim (DE); CHIN, Soo Yin, 67063 Ludwigshafen (DE); JOHANN, Thorsten, 67063 Ludwigshafen (DE)
(86) Internationale Anmeldenummer: PCT/EP2007/056940
(87) Internationale Veröffentlichungsnummer: WO 2008/006792

(56) Entgegenhaltungen:
- WO-A-2005/073157
- DE-B- 1 282 611
- US-A- 2 794 053
- US-A- 4 891 446

## Beschreibung

Die Erfindung betrifft ein Verfahren zur Dehydrierung von primären und sekundären Alkoholen zu den entsprechenden Aldehyden beziehungsweise Ketonen.

Acetaldehyd lässt sich durch katalytische Dehydrierung von Ethanol gewinnen. Großtechnisch durchgeführt wird, wie in Weissermel, Arpe: Industrielle Organische Chemie, 5. Auflage 1998, S. 186 - 187 beschrieben, die Dehydrierung an Silber- oder Kupferkatalysatoren sowie die oxidative Dehydrierung an Silberkatalysatoren in Gegenwart von Sauerstoff.

Die Dehydrierung wird beispielsweise an Kupferkatalysatoren, die mit Zn, Co oder Cr aktiviert sind, bei Temperaturen von 270 bis 300 °C durchgeführt. Bei einem Ethanol-Umsatz von 30 bis 50 % wird eine Selektivität zu Acetaldehyd von 90 bis 95 % erreicht. Als Nebenprodukte entstehen Ethylacetat, Crotonaldehyd, höhere Alkohole und Ethylen.

Bei der Oxidehydrierung in Gegenwart von Luft oder Sauerstoff liefert die simultane Verbrennung des gebildeten Wasserstoffs die notwendige Dehydrierwärme. Technisch wird die Oxidehydrierung an Silberkatalysatoren in Form von Drahtnetzen oder von Kristallschüttungen durchgeführt. Ethanol wird beispielsweise im Gemisch mit Luft bei 3 bar und 450 bis 550 °C über den Katalysator geleitet. Der Ethanol-Umsatz liegt bei 30 bis 50 %, die Selektivität zu Acetaldehyd bei 85 bis 95 %. Nebenprodukte sind Essigsäure, Ameisensäure, Ethylacetat, CO und CO₂.

Entsprechend wird Formaldehyd großtechnisch durch Dehydrierung oder Oxidehydrierung von Methanol an Kupfer- oder Silberkatalysatoren hergestellt.

Beim Wacker-Hoechst-Verfahren, nach dem 85 % der weltweiten Acetaldehyd-Produktion hergestellt werden, wird als Katalysator ein Zweikomponentensystem bestehend aus PdCl₂ und CuCl₂ eingesetzt. Als eigentlicher Katalysator fungiert PdCl₂, welches Ethylen stöchiometrisch selektiv zu Acetaldehyd oxidiert, wobei PdCl₂ zum Metall reduziert wird. Der CuCl₂-Cokatalysator oxidiert anschließend das metallische, nullvalente Pd in den zweiwertigen Zustand zurück. Das Verfahren wird als einstufiger oder zweistufiger Zweiphasen-Gas/Flüssig-Prozess durchgeführt, wobei Reaktion und Reoxidation des Platinkatalysators gemeinsam in einem einzigen Reaktor oder getrennt in zwei Reaktoren durchgeführt werden. Der einstufige Prozess wird bei 120 bis 130 °C bei 3 bar mit einem Umsatz von 35 bis 45 % durchgeführt, während der zweistufige Prozess bei 105 bis 110 °C bei 10 bar mit einem Umsatz von annähernd 100 % durchgeführt wird. Die Selektivitäten liegen um 94 % in beiden Fällen.

DE-A 1 282 611 offenbart Eisenmolybdat-Katalysatoren für die Oxidation von Methanol zu Formaldehyd.

US 4,891,446 offenbart die Dehydrierung von gesättigten aliphatischen primären Alkoholen zu den entsprechenden Aldehyden an einer Kupfer/Zink-Legierung in Gegenwart von Wasserstoff.

US 2,794,053 offenbart die Dehydrierung von sekundären aliphatischen Alkoholen zu Ketonen an einem Zinkoxid-Katalysator.

WO 2005/073157 offenbart die Dehydrierung von cyclischen und acyclischen Carbonylverbindungen, besonders bevorzugt von cyclischen und acyclischen Aldehyden und Ketonen, zu den entsprechenden α, β-ungesättigten Carbonylverbindungen an katalytisch aktiven Zusammensetzungen enthaltend Pd und Bi bzw. Pd, Rh und Bi sowie gegebenenfalls ein oder mehrere weitere Elemente. Erwähnt wird auch die Dehydrierung oxofunktionalisierter C₄-Kohlenwasserstoffe, wie Butanol, Buttersäure, Isobutanol, Isobuttersäure und Butyraldehyd.

Aufgabe der vorliegenden Erfindung ist es, geeignete katalytisch aktive Zusammensetzungen sowie alternative Verfahren für die Gasphasen-Dehydrierung von primären oder sekundären Alkoholen zu den entsprechenden Aldehyden beziehungsweise Ketonen bereitzustellen.

Gelöst wird die Aufgabe durch ein Verfahren zur Dehydrierung von primären oder sekundären Alkoholen mit 1 bis 12 C-Atomen zu den entsprechenden Aldehyden oder Ketonen, bei dem der Alkohol mit einer katalytisch aktiven Zusammensetzung umfassend eine Aktivkomponente der Formel

PdₐBi_{b}Y_{c}Z_{d}

in Kontakt gebracht wird, worin
Y ausgewählt ist aus der Gruppe bestehend aus Co, Rh, Pt, Ag, Au, und
Z ausgewählt ist aus der Gruppe bestehend aus Na, Cs, Mg, Ca, Ba, V, Cr, W, Fe, Ni, Cu, Sb, und
worin die Indizes a, b, c und d die Massenverhältnisse der jeweiligen Elemente untereinander angeben und
a = 0,1 - 3,
b = 0,1 - 3,
c = 0 - 3,
d = 0 - 1,
sind.

In einer Ausführungsform der Erfindung weist die Aktivkomponente die Formel PdₐBi_{b} auf, wobei
a = 0,1 - 3,
b = 0,1 - 3,
sind.

Bevorzugt ist c = 0,1 - 3, d. h. der Katalysator weist neben Palladium und Bismuth noch mindestens ein Element aus der Gruppe bestehend aus Co, Rh, Pt, Ag und Au und daneben optional noch ein oder mehrere weitere Elemente aus der Gruppe bestehend aus Na, Cs, Mg, Ca, Ba, V, Cr, W, Fe, Ni, Cu und Sb auf.

In einer bevorzugten Ausführungsform der Erfindung weist die Aktivkomponente die Formel PdₐBi_{b}Y_{c} auf, wobei Y = Au oder Rh ist, und wobei
a = 0,1 - -3,
b = 0,1 - 3,
c = 0,1 - 3,
sind.

In einer weiteren bevorzugten Ausführungsform weist die Aktivkomponente die Formel PdₐBi_{b}Rh_{c} auf, wobei
a = 0,1 - 3,
b = 0,1 - 3,
c = 0,1 - 3,
sind.

In einer weiteren bevorzugten Ausführungsform weist die Aktivkomponente die Formel PdₐBi_{b}RhₑY_{f}auf, wobei Y = Ag oder Pt ist, und
a = 0,1 - 3,
b = 0,1 - 3,
e = 0,1 - 3,
f = 0 - 1,
e + f ≤ 3
sind.

In einer weiteren bevorzugten Ausführungsform weist die Aktivkomponente die Formel PdₐBi_{b}Co_{c} auf, wobei
a = 0,1 - 3,
b = 0,1 - 3,
c = 0,1 -1,
sind.

Alle bisher aufgeführten katalytisch aktiven Zusammensetzungen können sowohl als Vollkatalysatoren als auch als Trägerkatalysatoren eingesetzt werden. Im Falle der Trägerkatalysatoren wird die Aktivkomponente der katalytisch aktiven Zusammensetzung auf ein geeignetes Trägermaterial aufgebracht.

Im Rahmen der vorliegenden Erfindung kann jedes dem Fachmann bekannte Trägermaterial verwendet werden. Auch können die Träger alle dem Fachmann bekannte Geometrien aufweisen, beispielsweise als Stränge, Ringe, Extrudate, Granalien, Granulate, Pulver, Tabletten usw. ausgebildet sein.

Bevorzugte Trägermaterialien sind ausgewählt aus der Gruppe bestehend aus Siliciumcarbiden, Siliciumnitriden, Carbonitriden, Oxonitriden, Oxocarbiden, Bismutoxid, Titanoxid, Zirkonoxid, Bornitrid, Aluminiumoxid, Silicaten, Alumosilicaten, zeolithischen sowie zeolithanalogen Materialien, Steatit, Aktivkohle, Metallnetzen, Edelstahlnetzen, Stahlnetzen sowie Mischungen aus zwei oder mehr der vorgenannten Trägermaterialien.

Besonders bevorzugt wird als Trägermaterial Steatit oder Siliciumcarbid eingesetzt.

Die genannten keramischen Träger können als Materialien mit großer Oberfläche, wie zum Beispiel größer als 100 m²/g, vorliegen. Bevorzugt werden jedoch im Rahmen der vorliegenden Erfindung Träger mit kleinen Oberflächen (kleiner als 100 m²/g), besonders bevorzugt Träger mit sehr kleinen Oberflächen (kleiner als 20 m²/g), eingesetzt. Ebenso können neben den reinen oxidischen, nitridischen oder carbidischen Trägern solche Trägermaterialien eingesetzt werden, denen basische Komponenten, beispielsweise Magnesiumoxid (MgO), Kalziumoxid (CaO), Bariumoxid (BaO) oder andere Alkali- oder Erdalkalikomponenten zugemischt sind oder die diese enthalten.

Im Rahmen der vorliegenden Erfindung werden besonders bevorzugt Trägermaterialien mit geringer Porosität (spezifische Oberfläche < 20 m²/g) beziehungsweise ohne Porosität eingesetzt.

Bevorzugt liegt die Gesamtbeladung des wenigstens einen Trägermaterials mit der Aktivkomponente im Bereich von 0,1 bis 20 Gew.-%, bevorzugt im Bereich von 8 bis 15 Gew.-% und weiterhin bevorzugt im Bereich von 0,1 bis 7 Gew.-% sowie besonders bevorzugt im Bereich von 0,5 bis 4 Gew.-%.

Wird im Rahmen der vorliegenden Erfindung Steatit als Trägermaterial eingesetzt, so wird insbesondere bevorzugt mit einer Gesamtbeladung an Aktivkomponente von 2 bis 4 Gew.-%, beispielsweise 3 Gew.-% gearbeitet.

Der Index a liegt in einem Bereich von 0,1 ≤ a ≤ 3, bevorzugt 0,5 ≤ a ≤ 2 und besonders bevorzugt 0,75 ≤ a ≤ 1,5. Index b liegt im Bereich von 0 ≤ b ≤ 3, bevorzugt 0,5 ≤ b ≤ 2 und besonders bevorzugt 0,75 ≤ b ≤ 1,5.

Der Index c liegt in einem Bereich von 0 ≤ c ≤ 3, bevorzugt 0,1 ≤ c ≤ 3 und besonders bevorzugt 0,5 ≤ c ≤ 2 und besonders bevorzugt 0,75 ≤ c ≤ 1,5. Index d liegt im Allgemeinen in einem Bereich von 0.5 d ≤ 1, bevorzugt 0 ≤ d ≤ 0,5 und besonders bevorzugt 0 ≤ d ≤ 0,1, wobei die Indizes die Massenverhältnisse der jeweiligen Elemente untereinander beziehungsweise in Gew.-% der jeweiligen Elemente, bezogen auf die Masse des Trägers, angeben.

Als besonders vorteilhaft haben sich katalytisch aktive Zusammensetzungen der nachstehenden Formeln erwiesen, welche eine Aktivkomponente der nachstehenden Formeln aufweisen:
- Pd_{0,5-1},₀Rh_{0.5-1,25}Bl_{1,25-1,75}Ag_{0,05-0,15}
- Pd₀,_{5-1,0}Rh_{1,0-1,5}Bi_{0,75-1,25}Pt_{0,01-0},₁
- Pd_{0,25-0,5}Rh_{1,75-2,5}Bl_{0,25-0,5}C°_{0,01-0,1}
- Pd_{0,5-1,25}Rh_{0,5-1,25}Bi_{0,75-1,5}Cr_{0,01-0,1}
- Pd_{1,0-1,75}Rh_{0,25-0,75}Bl_{0,75-1,5}Pt_{0,0-0},₁₅CO_{0,01-0,1}
- Pd_{1,0-1,75}Rh_{0,25-0,75}Bi_{0,75-1,5}Pt_{0,05-0,15}
- Pd_{0,5-1,0}Rh₁.₀₋₁,₇₅Bi_{0.5-1,25}Ag_{0,03-0.15}Ca_{0,02-0,1}
- Pd_{0,4-1,0}Rh_{1,0-1,75}Bi_{0,75-1,25}Ag_{0,03-0,15}
- Pd_{1,25-1,75}Rh_{1,25-1,75}CO_{0,005-0,02}
- Pd_{0,4-1,0}Rh_{1,0-1,75}Bi_{0,75-1,25}
- Pd_{0,15-2,25}Rh_{,0-2,5}Bi_{0,15-2,75}

Darin bedeuten die Indizes die Massenverhältnisse der einzelnen Elemente untereinander. Insbesondere bevorzugt sind dabei Zusammensetzungen der oben stehenden Formeln, für die zusätzlich a + b + c = 3 gilt.

Beispiele für katalytisch aktive Zusammensetzungen, die sich als besonders vorteilhaft erwiesen haben, weisen eine Aktivkomponente der folgenden Formel auf:
- Pd_{0,75}Rh_{0,75}Bi_{1,5}Ag_{0,1};
- Pd_{0,75}Rh_{1.25}Bi₁Pt_{0,05};
- Pd_{0,325}Rh_{2,25}Bi_{0,375}Co_{0,05};
- Pd_{0,85}Rh_{0,85}Bi_{1,25}Cr_{0,05};
- Pd_{1,4}Rh_{0,375}Bi_{1,125}Pt_{0,1}Co_{0,05};
- Pd_{1,4}Rh_{0,375}Bl_{1,125}Pt_{0,1};
- Pd_{0,}8Rh1_{,3}Bi_{0,85}Ag_{0,05}Ca_{0,05};
- Pd_{0,6}Rh_{1,33}Bi₁Ag_{0,08};
- Pd_{1,5}Bi_{1,5}Co_{0,01} oder
- Pd_{0,6}Rh_{1,33}Bi₁

Als besonders vorteilhaft haben sich beispielsweise katalytisch aktive Zusammensetzungen erwiesen, welche eine Aktivkomponente der folgenden Formel
- Pd_{0,75%}Rh_{0,75%}Bi_{1,5%}Ag_{0,1%};
- Pd_{0,75%}Rh_{1,25%}Bi_{1%}Pt_{0,05%};
- Pd_{0,325%}Rh_{2,25%}Bi_{0,375%}Co_{0,05%,}
- Pd_{0,85%}Rh_{0.85%}Bi_{1.25%}Cr_{0,05%;}
- Pd_{1,4%}Rh_{0,375%}Bi_{1,125%}Pt_{0,1%}Co_{0,05%};
- Pd_{1,4%}Rh_{0,375%}Bi_{1,125%}Pt_{0,1%;}
- Pd_{0.8%}Rh_{1,3%}Bi_{0,85%}Ag_{0.05}%Ca_{0.05%};
- Pd_{0,6%} Rh_{1,33%}Bi_{1%}Ag_{0,080%};
- Pd_{1,5%}Bi_{1,5%}Co_{0,01%} oder
- Pd_{0,6%} Rh_{1,33%}Bi_{1%};
aufgebracht auf wenigstens einem Trägermaterial, wie zuvor beschrieben, aufweisen, wobei die Indizes die Masseanteile (Gew.-%), bezogen auf das jeweilige Trägermaterial, angeben.

Als besonders geeignet für die Dehydrierung von Methanol zu Formaldehyd und von Ethanol zu Acetaldehyd haben sich weiterhin katalytisch aktive Zusammensetzungen der Formel
- Pd_{0,15-2,25}Rh_{0-2,5}Bi_{0,15-2,75}
- Pd_{0,1-1,0}Rh_{1,5-3,0}Bi_{0,1-1,0}
- Pd_{0,1-1,1}Rh_{1,0-2,6}Bi_{0,1-1,1}
- Pd_{0,1-1,1}Rh_{1,2-2,8}Bi_{0,1-1,1}
- Pd_{0,1-1,5}Rh_{1,0-2,99}Bi_{0,1-1,5}
- Pd_{1,0-2,0}Rh_{0,1-1,0}Bi_{0,5-2,0}
erwiesen. Diese liegen insbesondere auf Steatit als Träger vor.

### Beispiele sind

- Pd₀₃₇₅Rh_{2,25}Bi_{0,375}
- Pd_{0,6}Rh_{1,8}Bi_{0,6}
- Pd_{0,45}Rh_{2,1}Bi_{0,45}
- Pd_{0,5}Rh_{2,0}Bi_{0,5}
- Pd_{1,5}Rh_{0,375}Bi_{1,125}

Als besonders geeignet für die Dehydrierung von Methanol zu Formaldehyd und von Ethanol zu Acetaldehyd haben sich weiterhin katalytisch aktive Zusammensetzungen der Formeln
- Pd_{0,4-1,0}Rh_{1,0-1,75}Bi_{0,75-1,25}Ag_{0,03-0,15}
- Pd_{1,25-1,75}Rh_{1,25-1,75}Co_{0,005-0,02}
- Pd_{0,2-1,0}Bi_{0,6-1,4}Rh_{0,93-1,73}Ag_{0,01-0,20}
- Pd_{1,0-2,0}Bi_{1,0-2,0}Co_{0,005-0,15}
- Pd_{1,0-2,0}Bi_{1,0-2,0}Pt_{0,05-0,15}
- Pd_{0,7-1,7}Rh_{0,3-1,5}Bi_{0,3-1,5}CO_{0,05-0,15}
erwiesen. Diese liegen insbesondere auf Steatit als Träger vor.

Beispiele sind
- Pd₀,₆Bi₁Rh_{1,33}A9_{0,08}
- Pd_{1,5}Bi_{1,5}Co_{0,01}
- Pd_{1,5}Bi_{1,5}Pt_{0,1}
- Pd_{1,2}Rh_{0,9}Bi_{0,9}Co_{0,1}

Als besonders geeignet für die Dehydrierung von Methanol zu Formaldehyd und von Ethanol zu Acetaldehyd haben sich weiterhin katalytisch aktive Zusammensetzungen der Formeln
- Pd_{0,5-2,0}Rh_{0,1-1,1}Bi_{0,5-2,0}
- Pd_{1,0-2,0}Rh_{0,1-1},₀Bi_{0.5-2,0}
- Pd_{1,0-2,5}Rh_{0,01-0,5}Bi_{0,5-1,75}
- Pd_{0,1-1,5}Rh_{0,5-1,57}Bi_{1,25}Pt_{0,001-0,1}
erwiesen. Diese liegen insbesondere auf Steatit als Träger vor.

Beispiele sind
- Pd_{1,2}Rh_{0,6}Bi_{1,2}
- Pd_{1,5}Rh_{0,375}Bi_{1,125}
- Pd_{1,8}Rh_{0,15}Bi_{1,05}
- Pd_{0,75}Rh₁Bi_{1,25}Pt_{0,05}

In einer besonders bevorzugten Ausführungsform werden die oben aufgeführten Aktivkomponenten auf Steatit, Siliciumcarbid oder eine Mischung aus beiden als Trägermaterial aufgebracht.

Grundsätzlich können erfindungsgemäß eingesetzte Vollkatalysatoren nach allen dem Fachmann bekannten Herstellungsverfahren für Vollkatalysatoren hergestellt werden. Die bevorzugte Herstellungsweise ist die Co-Fällung. Dabei werden eine, zwei oder mehrere Elemente aus der Gruppe der Aktivkomponenten als wässrige Salzlösungen gemischt und dann in Form ihrer Hydroxide oder Carbonate gemeinsam gefällt. Es entsteht dabei eine amorphe oder auch kristalline Ausfällung oder ein Gel. Gegebenenfalls kann der entstehende Niederschlag salzfrei gewaschen werden. Das erhaltene Produkt wird in einem nächsten Verfahrensschritt getrocknet. Gegebenenfalls kann der getrocknete Feststoff zur verbesserten Homogenisierung des Produkts zusätzlich gemahlen werden. Ebenso kann der Feststoff gegebenenfalls noch geformt werden, wobei im Rahmen der Formgebung das vorliegende Produkt gegebenenfalls durch Kneten plastifiziert und zur Strängen extrudiert oder auch nach Beimengung von Hilfsstoffen zu Tabletten verpresst werden kann. Im Anschluss daran wird das getrocknete Produkt kalziniert.

Das kalzinierte Produkt kann gegebenenfalls aktiviert und gegebenenfalls auf seine katalytischen Eigenschaften wie Selektivität und Aktivität sowie Stabilität getestet werden. Die Testung kann nach allen dem Fachmann bekannten Methoden, wie beispielsweise dem probeweisen Einsatz eines Katalysators in ausgewählten Reaktionen und die Analyse seiner katalytischen Eigenschaften, erfolgen.

Somit weist das Verfahren zur Herstellung der Vollkatalysatoren die folgenden Schritte auf:
(i) Ausfällen der wenigstens einen Aktivkomponente aus einer ihre Salze enthaltenden Lösung;
(ii) Trocknung des in Schritt (i) hergestellten Produkts;
(iii) Kalzinierung des in Schritt (ii) getrockneten Produkts;
(iv) gegebenenfalls Testung des in Schritt (iii) kalzinierten Produkts.

Bevorzugt werden im Rahmen der vorliegenden Erfindung die Imprägnierung des Trägerkörpers unterhalb der Wasseraufnahme des Trägers ("incipient wetness") oder die Adsorption aus überstehender Lösung oder das Aufbringen dünner Schichten auf keramische Trägermaterialien als Synthesewege zur Herstellung von Trägerkatalysatoren eingesetzt.

Im Allgemeinen werden im Rahmen aller genannten Verfahren die Elemente der Aktivkomponente als thermisch unbeständige Salze wie beispielsweise Nitrate, Acetate, Carbonate oder Hydroxide eingesetzt. Im Rahmen des Imprägnierens aus überstehender Lösung wird der Träger in die Lösung, welche die Elemente der jeweilig Aktivkomponente in Form ihrer Anionen aufweist, eingetaucht und unter genau definierten Bedingungen bezüglich der Konzentration, Vermischung, Temperatur und Zeit behandelt. Um die Effektivität der Imprägnierung zu erhöhen, kann gegebenenfalls die Luft in den Trägerporen durch Evakuierung entfernt oder der Träger vor dem Imprägnieren begast werden. Dem Imprägnierungsschritt schließt sich in der Regel ein Trocknungs- und Kalzinierungsschritt an.

Bei dem Syntheseweg über das Aufbringen dünner Schichten auf keramische Trägermaterialien können die jeweiligen Precursorlösungen nacheinander einzeln oder bevorzugt als Mischung gemeinsam auf die Träger aufgebracht werden. In diesem Fall werden bevorzugt die thermisch unbeständigen Anionen der jeweiligen Elemente, welche die Aktivkomponente gemäß obiger Ausführungen aufweist, eingesetzt. Das Aufbringen kann durch einfache Abgabe aus einer Pipette, aber auch durch Aufsprühen, Sprüh-Gefriertrocknung sowie alle anderen dem Fachmann in diesem Zusammenhang bekannten Techniken durchgeführt werden. Ebenso ist es möglich, mit Hilfe der Sprüh-Gefriertrocknung, wie in DE 102 11 260.6 beschrieben, dünne Schichten der jeweiligen Elemente der katalytisch aktiven Zusammensetzung auf einen gewünschten Träger aufzubringen.

Nach dem Aufbringen der Precursorlösung schließt sich in der Regel ein Trocknungsschritt an. Im Rahmen dieses Trocknungsschritts werden die Materialien zwischen 30 Minuten und 24 Stunden bei Temperaturen zwischen 40 °C und 150 °C getrocknet. Bevorzugt werden die Materialien 3 Stunden bei 80 °C getrocknet. Ebenfalls bevorzugt ist die Gefriertrocknung der Materialien im Vakuum beziehungsweise bei vermindertem Druck.

An den Schritt der Trocknung schließt sich in der Regel ein Kalzinierungsschritt an. Unter Kalzinierung wird im Allgemeinen eine Wärmebehandlung in oxidierender Atmosphäre bei Temperaturen in der Regel oberhalb der späteren Einsatztemperaturen der katalytisch aktiven Zusammensetzung verstanden. Im Rahmen der vorliegenden Erfindung werden dabei die Materialien zwischen 1 und 100 Stunden mit einer Aufheizrate im Bereich von 0,25 °C/min bis 10 °C/min auf eine Endtemperatur zwischen 200 °C und 1200 °C aufgeheizt und bei der gewählten Temperatur zwischen 30 min und 150 Stunden belassen. Im Rahmen der vorliegenden Erfindung wird bevorzugt eine Rampe von 3 °C/min, eine Endtemperatur von 550 °C sowie eine Haltezeit von 3 Stunden bevorzugt. Als Kalzinierungsatmosphäre kommen Luft, N₂, Formiergas (H₂ in N₂, zum Beispiel 5% H₂ in N₂), Reaktivgase (Cl₂, NH₃ und andere), oder Vakuum in Frage. Bevorzugt wird die Kalzinierung unter Luft oder N₂ durchgeführt.

Demgemäß weist das bevorzugte Verfahren zur Herstellung einer katalytisch aktiven Zusammensetzung umfassend wenigstens eine Aktivkomponente, aufgebracht auf wenigstens einem Trägermaterial, wenigstens die folgenden Schritte auf:
(α) Aufbringen einer Lösung aufweisend wenigstens eine Aktivkomponente auf wenigstens ein Trägermaterial;
(β) Trocknung des in Schritt (α) hergestellten Produkts;
(χ) Kalzinierung des in Schritt (β) getrockneten Produkts;
(δ) gegebenenfalls Testung des in Schritt (χ) kalzinierten Produkts.

In einem weiteren Schritt können die katalytisch aktiven Zusammensetzungen auf ihre katalytischen Eigenschaften getestet werden.

Die Testung der im Rahmen dieser Erfindung hergestellten katalytisch aktiven Zusammensetzungen erfolgt durch Einbau von beispielsweise wenigstens 1 ml des zu testenden Materials in einen dem Fachmann bekannten Edelstahlreaktor. Nach der katalytischen Umsetzung innerhalb des Reaktors kann die sich anschließende Produktgasanalyse mit allen dem Fachmann dafür bekannten Analysemethoden, beispielsweise mit einem GC/MS mit einer HP-5-MS-Säule zur Trennung und Bestimmung der Produkte und Edukte durchgeführt werden.

Bevorzugt wird das erfindungsgemäße Verfahren zur Dehydrierung von Methanol zu Formaldehyd und von Ethanol zu Acetaldehyd eingesetzt. Darüber hinaus kann es zur Dehydrierung von 1-Propanol zu Propionaldehyd, von iso-Propanol zu Aceton, von 1-Butanol zu Butyraldehyd, von 2-Butanol zu Methylethylketon, von iso-Butanol zu iso-Butyraldehyd sowie zur Dehydrierung der isomeren primären und sekundären Pentanole, Hexanole, Heptanole, Octanole, Nonanole, Decanole, Undecanole und Dodecanole zu den entsprechenden Aldehyden beziehungsweise Ketonen eingesetzt werden. Bevorzugt dehydriert werden die C₁-C₆-Alkanole, insbesondere die genannten C₁-C₄-Alkanole.

Besonders bevorzugt wird die Dehydrierung in Gegenwart von Sauerstoff durchgeführt.

In einer weiterhin bevorzugten Ausführungsform der vorliegenden Erfindung wird die Dehydrierung von Kohlenwasserstoffen unter Verwendung einer katalytisch aktiven Zusammensetzung, wie oben beschrieben, in Gegenwart von Sauerstoff und Wasserdampf durchgeführt.

Der Sauerstoffgehalt bei den Dehydrierungen, welche wenigstens in Gegenwart von Sauerstoff oder Sauerstoff und Wasser durchgeführt werden, liegt im Rahmen der vorliegenden Erfindung im Verhältnis zum Gesamtvolumen der zugeführten Edukte im Bereich von 1 Vol.-% bis 50 Vol.-%, bevorzugt im Bereich von 1 Vol.-% bis 30 Vol.-% und besonders bevorzugt im Bereich von 1 Vol.% bis 10 Vol.-% oder von 20 bis 30 Vol.-%.

Der Wassergehalt bei Dehydrierungen, welche wenigstens in Gegenwart von Sauerstoff und Wasserstoff durchgeführt werden, liegt im Verhältnis zum Gesamtvolumen der zugeführten Edukte im Bereich bis zu 50 Vol.-%, bevorzugt im Bereich von 1 Vol.-% bis 35 Vol.-%, besonders bevorzugt im Bereich von 5 Vol.-% bis 25 Vol.-%. Gegebenenfalls kann bei den vorgeschriebenen Dehydrierungen Stickstoff als Balancegas zugeführt werden.

Der Alkoholgehalt bei Dehydrierungen, welche wie oben beschrieben durchgeführt werden, liegt bezogen auf das Gesamtvolumen der zugeführten Edukte, im Bereich von 0 bis 90 Vol.-%, bevorzugt im Bereich von 0,01 bis 25 Vol.-%, besonders bevorzugt im Bereich von 0,1 bis 30 Vol.-%, beispielsweise von 0,1 bis 4 Vol.-% oder von 15 bis 25 Vol.-%.

Das Molverhältnis Alkohol zu Sauerstoff liegt im Rahmen der vorliegenden Erfindung im Allgemeinen in einem Bereich von 3 : 1 bis 1 : 20, bevorzugt in einem Bereich von 1 : 1 bis 1 : 7, besonders bevorzugt in einem Bereich von 1 : 2 bis 1 : 5.

Das Molverhältnis Alkohol zu Wasser liegt im Rahmen des erfindungsgemäßen Verfahrens in einem Bereich von 3 : 1 bis 1 : 50, bevorzugt in einem Bereich von 1 : 5 bis 1 : 40, besonders bevorzugt in einem Bereich von 1 : 10 bis 1 : 30.

Die erfindungsgemäßen eingesetzten katalytischen Zusammensetzungen zeichnen sich dadurch aus, dass sie eine Dehydrierung schon bei relativ niedrigen Temperaturen von deutlich unterhalb 400 °C ermöglichen. Die Katalysatoraktivität bleibt über lange Zeit praktisch unverändert, so dass eine Reaktivierung nur selten erforderlich ist.

Die Reaktionstemperaturen liegen in einem Bereich zwischen im Allgemeinen 150 °C und 450 °C, bevorzugt zwischen 150 und 350 °C, besonders bevorzugt zwischen 200 und 300 °C und speziell zwischen 210 und 260 °C.

Die jeweilige Gasbelastung (GHSV) liegt dabei in einem Bereich zwischen 100 h⁻¹ und 100 000 h⁻¹, bevorzugt zwischen 1000 h⁻¹ und 10 000 h⁻¹.

Für die Dehydrierung von Ethanol zu Acetaldehyd haben sich folgende Reaktionsbedingungen unter anderem als besonders günstig erwiesen: Die Umsetzung findet bei einer Temperatur zwischen 200 und 300 °C, bevorzugt zwischen 210 und 260 °C statt. Die Belastung (GHSV) liegt zwischen 100 und 50 000 h⁻¹, bevorzugt zwischen 1000 und 10 000 h⁻¹. Der Ethanol-Gehalt im Einsatzgasstrom (Feed) liegt zwischen 0,1 Vol.-% und 15 Vol.-%, bevorzugt zwischen 1 Vol.-% und 10 Vol.-%. Das Ethanol : Sauerstoff-Molverhältnis im Einsatzgasstrom beträgt von 10 : 1 bis 1 : 5. Der Einsatzgasstrom kann bis zu 20 Vol.-% Wasser, vorzugsweise 0,1 bis 10 Vol.-% Wasser enthalten.

Der Dehydrierungskatalysator kann im Reaktor fest angeordnet oder z. B. in Form eines Wirbelbettes eingesetzt werden und eine entsprechende Gestalt haben. Geeignet sind z. B. Formen wie Splitt, Tabletten, Monolithe, Kugeln oder Extrudate (Stränge, Wagenräder, Sterne, Ringe).

Eine geeignete Reaktorform ist der Festbettrohr- oder Rohrbündelreaktor. Bei diesen befindet sich der Katalysator als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z. B. ein Kohlenwasserstoff wie Methan, verbrannt wird oder ein Wärmeträgermedium (Salzbad, Wälzgas etc.) eingesetzt wird. Es kann auch eine elektrische Beheizung der Reaktionsrohre mit Heizmanschetten erfolgen. Übliche Reaktionsrohr-Innendurchmesser betragen etwa 1 bis 15 cm. Ein typischer Dehydrierrohrbündelreaktor umfasst ca. 10 bis 32000 Reaktionsrohre.

Im Allgemeinen erfolgt die Regenerierung der katalytisch aktiven Zusammensetzung durch Abbrennen des auf der Katalysatoroberfläche abgelagerten Koks in Gegenwart von Sauerstoff. Hierzu wird Luft oder Sauerstoff, der mit Inertgasen verdünnt sein kann, zu dem Einsatzgasstrom, der die zu dehydrierende Verbindung enthält, zugegeben, wobei deren Gehalt in dem Gasstrom während der Regenerierung auf 0 Vol.-% reduziert werden kann. Die Regenerierung wird bei einer Temperatur von im Allgemeinen 200 bis 400 °C durchgeführt.

In einer Variante der Regenerierung werden die erfindungsgemäßen katalytisch aktiven Zusammensetzung zunächst bei Temperaturen von 200 bis 400 °C, bevorzugt von 250 bis 350 °C in einem Zeitraum von 1 min bis 100 h, bevorzugt 10 min bis 24 h, besonders bevorzugt 30 min bis 1,5 h abgebrannt, so dass der auf der Katalysatoroberfläche abgelagerte Koks zu Kohlendioxid abbrennt.

Bevorzugt wird der Abbrand im Rahmen der vorliegenden Erfindung bei einer Temperatur um 350 °C in einer Atmosphäre von etwa 1 % Sauerstoff in Stickstoff, bevorzugt 5 % Sauerstoff in Stickstoff, besonders bevorzugt etwa 10 % Sauerstoff in Stickstoff durchgeführt.

Nach dem Abbrand wird die die katalytisch aktive Zusammensetzung umgebende Atmosphäre mit Stickstoff sauerstofffrei gespült.

Als dritter Schritt im Rahmen der Regenerierung erfolgt eine Wasserstoffbehandlung der katalytisch aktiven Zusammensetzung. Diese wird bevorzugt bei Temperaturen im Bereich von 220 bis 280 °C, besonders bevorzugt 250 bis 270 °C in Gegenwart von Formiergas durchgeführt. Besonders bevorzugt wird dabei Formiergas mit einer Zusammensetzung von etwa 3 % Wasserstoff in Stickstoff verwendet. Die Wasserstoffbehandlung erfolgt in einem Zeitraum von 1 min bis 100 h, bevorzugt 10 min bis 24 h, besonders bevorzugt 30 min bis 1,5 h.

Im Anschluss daran wird die die katalytisch aktive Zusammensetzung umgebende Atmosphäre wasserstofffrei gespült.

Im Folgenden wird die vorliegende Erfindung durch Beispiele verdeutlicht.

Die Beispiele zeigen die Herstellung verschiedener katalytisch aktiver Verbindungen sowie deren Testung auf katalytische Eigenschaften.

Soweit im Rahmen der einzelnen Beispiele nichts Gegenteiliges angegeben ist, wird für die Testung 1 ml der jeweilig katalytisch aktiven Zusammensetzung verwendet.

### Beispiele

### Allgemeine Herstellvorschrift für die Katalysatoren

Als Träger wird spherisches Steatit (Ceram Tek, 4 - 5 mm, 83577) verwendet. In allen Fällen wurde als Pd-Precursor Pd(NO₃)₂ · H₂O, als Bi-Precursor Bi(NO₃)₃ · 5H₂O und als Rh-Precursor eine Rh-Lösung enthaltend 13,7 Gew.-% Rh verwendet. Wegen des Löslichkeitsverhaltens der Pd- und Bi-Vorläufer wurde 65 gew.-%ige Salpetersäure (HNO₃ als Lösungsmittel für diese beiden Vorläufer verwendet. Während der Herstellung von Materialien enthaltend eines dieser Metalle oder beide Metalle und zusätzlich Rh wurden die Vorläufer zuerst in leicht erwärmter HNO₃ gelöst und bis zur vollständigen Auflösung gerührt, gefolgt von der Zugabe der Rh-Lösung.

Das resultierende Gemisch wurde anschließend mit einer Pipette tropfenweise zu dem Steatit zugegeben, wobei mit einem Magnetrührer gerührt wurde. Nach Zugabe der gesamten Lösung wurde 2 Minuten weiter gerührt, um eine homogene Beschichtung des Steatit-Trägers mit den Katalysatormetall-Vörläufern zu gewährleisten. Im Falle der Verwendung von überschüssiger Lösung wurde der Steatit gerührt und leicht erwärmt, bis die gesamte Lösung verdunstet war. Nach der Imprägnierung wurden die Katalysatoren bei 80 °C im Stickstoffstrom 16 h lang getrocknet und anschließend 3 h lang bei 550 °C im Stickstoffstrom calciniert, wobei die Temperaturrampe 5 °C/min betrug.

### Allgemeine Testvorschrift

Zum Test der Katalysatoren wurden 42,6 ml des jeweiligen Katalysators in einen Edelstahl-Rohrreaktor eingefüllt, welcher in ein Salzbad eintauchte. Die Reaktionstemperatur wurde über die Salzbadtemperatur geregelt. Der Edelstahlreaktor selbst verhielt sich unter den Reaktionsbedingungen inert. Die Analyse der Reaktionsprodukte erfolgte gaschromatographisch unter Verwendung eines GC-Systems der Agilent 6890-Serie. Die Gasphasenprodukte wurden mit einer Crompack 7574-Kapillarsäule und einem Online-Gas-Analysator (Ultramat 23 von Siemens), der mit CO-, CO₂- und O₂-Sensoren ausgestattet war, analysiert. Die flüssigen Produkte wurden mittels einer Restek 10193-Kapillarsäule analysiert.

Vor dem Test wurden alle Katalysatoren im Stickstoffstrom 20 Minuten lang bei der Reaktionstemperatur erhitzt.

### Beispiel 1: Pd auf Steatit

Die Imprägnierlösung enthielt 1,63 g Pd (NO₃)₂ · H₂O in 8 ml HNO₃.

Die oxidative Dehydrierung von Ethanol wurde bei einer Gasbelastung von 7300 h⁻¹ (2,6 % Ethanol, 2,6 % O₂ und 6,4 % H₂O in Stickstoff) bei einer Temperatur von 220 °C durchgeführt. Der Ethanol-Umsatz betrug 12,5 %, die Selektivität zu Acetaldehyd 66,5 %, entsprechend einer Acetaldehydausbeute von 8,3 %.

Bei Durchführung der Reaktion bei 260 °C mit 3,8 % Ethanol, 3,7 % O₂ und 7,4 % H₂O in Stickstoff wurde ein Ethanol-Umsatz von 19,2 % mit einer Selektivität zu Acetaldehyd von 20,0 % erhalten, entsprechend einer Acetaldehyd-Ausbeute von 11,9 %.

### Beispiel 2: Bi auf Steatit (0,375 Gew.-%)

Die Imprägnierlösung enthielt 1,75 g Bi (NO₃)₃ · 5 H₂O in 8 ml HNO₃. Die oxidative Dehydrierung von Ethanol wurde bei einer Gasbelastung von 7300 h⁻¹ mit 3,8 % Ethanol, 3,7 % O₂ und 7,3 % H₂O in Stickstoff bei 220 und 260 °C durchgeführt. Ein Ethanol-Umsatz wurde nicht beobachtet.

### Beispiel 3: Pd-Bi auf Steatit (Gesamtbeladung: 0,75 Gew.-%)

Die Imprägnierlösung enthielt 1,64 Gew.-% Pd(III)nitrat, entsprechend 0,375 Gew.-% Metall, und 1,75 Gew.-% Bi(III)t)nitrat, entsprechend 0,375 Gew.-% Metall. Die oxidative Dehydrierung von Ethanol wurde bei einer Gasbelastung von 7300 h⁻¹ mit 3,8 % Ethanol, 3,7 O₂ und 7,3 % H₂O in Stickstoff durchgeführt. Bei 220 ° C wurde ein Ethanol-Umsatz von 48,4 % und eine Selektivität zu Acetaldehyd von 9,6 % erzielt, entsprechend einer Ausbeute von 4,6 %.

### Beispiel 4: Rh auf Steatit

Die Imprägnierlösung enthielt 33,6 g Rhodiumlösung, entsprechend 4,6 g Rh. Die oxidative Dehydrierung von Ethanol wurde bei einer Gasbelastung von 7300 h⁻¹ mit 3,8 % Ethanol, 3,7 % O₂ und 7,3 % H₂O in Stickstoff durchgeführt. Bei 220 °C wurde ein Umsatz von 41,9 % mit einer Selektivität zu Acetaldehyd von 10,6 %, entsprechend einer Ausbeute von 4,4 %, erzielt.

### Beispiel 5: Rh-Bi auf Steatit (Gesamtbeladung: 2,63 Gew.-%)

Die Imprägnierlösung enthielt 0,77 g des Bi-Precursors in 3 ml HNO₃ und 33,7 g der Rh-Precursorlösung. Die oxidative Dehydrierung von Ethanol wurde bei einer Gasbelastung von 7300 h⁻¹mit 3,8 % Ethanol, 3,7 % O₂ und 7,3 % H₂O in Stickstoff durchgeführt. Bei 220 °C wurde ein Umsatz von 10,1 % erhalten. Bei 280 °C wurde ein Umsatz von 85,7 % erreicht.

### Beispiel 6: Pd-Rh auf Steatit

Die Imprägnierlösung enthielt 0,37 g des Pd-Precursors in 3 ml HNO₃ und 33,7 g der Rh-Precursorlösung. Die oxidative Dehydrierung von Ethanol wurde bei einer Gasbelastung von 7300 h⁻¹ mit 3,8 % Ethanol, 3,7 % O₂, 7,3 % H₂O in Stickstoff durchgeführt. Bei 220 °C wurde ein Ethanol-Umsatz von 48,4 % bei einer Selektivität zu Acetaldehyd von 9,6 %, entsprechend einer Ausbeute von 4,6 %, erhalten.

### Beispiel 7: Pd-Bi-Co auf Steatit (Gesamtbeladung: 3 Gew.-%)

Die Imprägnierlösung enthielt 6,7 g, entsprechend 1,5 Gew.-% des Pd-Precursors, 7,036 g, entsprechend 1,5 Gew.-% des Bi-Precursors und 0,1 g, entsprechend 0,01 Gew.-% Co(NO₃)₂. Die oxidative Dehydrierung von Ethanol wurde bei einer Gasbelastung von 7300 h⁻¹ mit 3,8 % Ethanol, 3,7 % O₂, 7,3 % H₂O in Stickstoff durchgeführt. Die Ergebnisse der Versuche sind in Tabelle 1 zusammengefasst.

Tabelle 1

| Temperatur [°C] | Umsatz [%] | Ausbeute [%] | Selektivität [%] |
|---|---|---|---|
| 210 | 76,6 | 68,4 | 89,3 |
| 220 | 98,0 | 78,5 | 80,1 |
| 260 | 98,4 | 78,0 | 79,3 |

### Beispiel 8: Pd-Bi-Rh auf Steatit (Gesamtbeladung: 3 Gew.-%)

Die Imprägnierlösung enthielt 1,67 g des Pd-Precursors, entsprechend 0,38 Gew.-%, 1,83 g des Bi-Precursors, entsprechend 0,38 Gew.-%, und 33,9 g Rh(NO₃)₃-Lösung, entsprechend 2,25 Gew.-%.

Die Versuchsbedingungen und Versuchsergebnisse sind in der nachstehenden Tabelle wiedergegeben.

**Tabelle 2**

| Temperatur | GHSV | EtOH | O₂ | H₂O | Umsatz | Ausbeute | Selektivität |
|---|---|---|---|---|---|---|---|
| [°C] | [h⁻¹] | [%] | [%] | [%] | [%] | [%] | [%] |
| 220 | 7316 | 3,8 | 4,0 | 7,4 | 93,8 | 87,2 | 93,0 |
| 240 | 7316 | 3,8 | 3,7 | 7,4 | 99,5 | 82,6 | 83,0 |
| 260 | 7316 | 3,8 | 3,7 | 7,4 | 99,5 | 78,2 | 78,6 |

### Beispiel 9: Abhängigkeit der katalytischen Aktivität von der Sauerstoffkonzentration

Es wurde das Material aus Beispiel 8 bei einer Gasbelastung von 7316 h⁻¹ mit 4,96 % Ethanol, 3,5 % H₂O und verschiedenen Sauerstoff-Konzentrationen in Stickstoff bei 220 °C getestet. Die Ergebnisse sind in der nachstehenden Tabelle wiedergegeben.

**Tabelle 3**

| O₂/EtOH-Molverhältnis | Umsatz [%] | Ausbeute [%] | Umsatz [%] |
|---|---|---|---|
| 0,4 | 73,1 | 73,0 | 100,0 |
| 0,6 | 94,5 | 89,5 | 91,8 |
| 0,8 | 99,9 | 81,8 | 82,0 |
| 1,0 | 100,0 | 73,9 | 73,9 |

### Beispiel 10: Abhängigkeit der katalytischen Aktivität von der H₂O-Konzentration

Das Material aus Beispiel 8 wurde bei einer Gasbelastung von 7316 h⁻¹ mit 3,8 % Ethanol und einem O₂-/EtOH-Verhältnis von 1,1 : 1 und verschiedenen Wasserdampf-Konzentrationen in Stickstoff bei 220 °C getestet. Die Ergebnisse sind in der nachstehenden Tabelle zusammengefasst.

**Tabelle 4**

| H₂O [%] | Umsatz [%] | Ausbeute [%] | Selektivität [%] |
|---|---|---|---|
| 0,73 | 97,7 | 90,6 | 92,8 |
| 4,0 | 95,0 | 89,9 | 94,7 |
| 7,4 | 93,8 | 87,2 | 93,0 |

### Beispiel 11: Abhängigkeit von der Ethanol-Konzentration

Das Material aus Beispiel 8 wurde bei einer Gasbelastung von 7316 h⁻¹ mit 1 % H₂O und einem Verhältnis O₂/EtOH von 0,72 : 1 in Stickstoff bei 220 °C getestet. Die Ergebnisse sind in der nachstehenden Tabelle zusammengefasst.

**Tabelle 5**

| EtOH [%] | Umsatz [%] | Ausbeute [%] | Selektivität [%] |
|---|---|---|---|
| 4,5 | 99,8 | 88,0 | 88,2 |
| 5,6 | 99,4 | 85,4 | 86,0 |
| 6,5 | 98,3 | 85,7 | 87,1 |
| 7,4 | 98,0 | 85,1 | 86,8 |

## Patentansprüche

1. Verfahren zur Dehydrierung von primären oder sekundären Alkoholen mit 1 bis 12 C-Atomen zu den entsprechenden Aldehyden oder Ketonen, bei dem der Alkohol mit einer katalytisch aktiven Zusammensetzung umfassend eine Aktivkomponente der Formel
PdₐBi_{b}Y_{c}Z_{d}
in Kontakt gebracht wird, worin
Y ausgewählt ist aus der Gruppe bestehend aus Co, Rh, Pt, Ag, Au, und
Z ausgewählt ist aus der Gruppe bestehend aus Na, Cs, Mg, Ca, Ba, V, Cr, W, Fe, Ni, Cu, Sb
worin die Indizes a, b, c und d die Massenverhältnisse der jeweiligen Elemente untereinander angeben, mit
a = 0,1 - 3,
b = 0,1 - 3,
c = 0 - 3,
d = 0 - 1.

2. Verfahren nach Anspruch 1, **dadurch gekennzeichnet, dass** die Aktivkomponente die Formel
PdₐBi_{b}Y_{c}
aufweist, wobei Y = Au oder Rh ist, mit
a = 0,1 - 3,
b=0,1 -3,
c = 0 - 3.

3. Verfahren nach Anspruch 1 oder 2, wobei c mindestens 0,1 ist.

4. Verfahren nach Anspruch 1, **dadurch gekennzeichnet, dass** die Aktivkomponente die Formel
PdₐBi_{b}Rh_{c}
aufweist, mit
a = 0,1 - 3,
b = 0,1 - 3,
c = 0,1 - 3.

5. Verfahren nach Anspruch 1, **dadurch gekennzeichnet, dass** die Aktivkomponente die Formel
PdₐBi_{b}RhₑY_{f}
aufweist, wobei Y = Ag oder Pt ist, mit
a = 0,1 - 3,
b=0,1 -3,
e = 0,1 - 3,
f=0-1,
e + f ≤ 3.

6. Verfahren nach Anspruch 1, **dadurch gekennzeichnet, dass** die Aktivkomponente die Formel
PdₐBi_{b}Co_{c}
aufweist, mit
a = 0,1 - 3,
b=0,1 -3,
c = 0,1 - 1.

7. Verfahren nach Anspruch 1, **dadurch gekennzeichnet, dass** die Aktivkomponente eine der nachstehenden Formeln
- Pd_{0,5-1,0}Rh_{0,5-1,2}5Bi_{1,25-1,75}Ag_{0,05-0,15}
- Pd_{0,5-1,0}Rh_{1,0-1,5}Bi_{0,74-1,25}Pt_{0,01-0,1}
- Pd_{0,25-0,5}Rh_{1,75-2,5}Bi_{0,25-0,5}Co_{0,01-0,1}
- Pd_{0,5-1,25}Rh_{0,5-1,25}Bi_{0,75-1,5}Cr_{0,01-0,1}
- Pd_{1,0-1,75}Rh_{0,25-0,75}Bi_{0,75-1,5}Pt_{0,0-0,15}Co_{0,01-0,1}
- Pd_{1,0-1,75}Rh_{0,25-0,75}Bi_{0,75-1,5}Pt_{0,05-0,15}
- Pd_{0,5-1,0}Rh_{1,0-1,75}Bi_{0,5-1,25}Ag_{0,03-0,15}Ca_{0,02-0,1}
- Pd_{0,4-1,0}Rh_{1,0-1,75}Bi_{0,75-1,25}Ag_{0,03-0,15}
- Pd_{1,25-1,75}Bi_{1,25-1,75}Co_{0,005-0,02}
- Pd_{0,4-1,0}Rh_{1,0-1,75}Bi_{0,75-1,25}
- Pd_{0,15-2,25}Rh_{0-2,5}Bi_{0,15-2,75}
aufweist.

8. Verfahren nach einem der Ansprüche 1 bis 7, **dadurch gekennzeichnet, dass** die Aktivkomponente auf einem Trägermaterial ausgewählt aus der Gruppe bestehend aus Siliciumcarbiden, Siliciumnitriden, Carbonitriden, Oxonitriden, Oxocarbiden, Bismutoxid, Titanoxid, Zirkonoxid, Bornitrid, Aluminiumoxid, Silicaten, Alumosilicaten, zeolithischen sowie zeolithanalogen Materialien, Steatit, Aktivkohle, Metallnetze, Edelstahlnetze, Stahlnetze sowie Mischungen aus zwei oder mehr der vorgenannten Trägermaterialien vorliegt.

9. Verfahren nach einem der Ansprüche 1 bis 8, **dadurch gekennzeichnet, dass** der zu dehydrierende Alkohol ausgewählt ist aus Methanol, Ethanol, n-Propanol und Isopropanol.

10. Verfahren nach einem der Ansprüche 1 bis 9, **dadurch gekennzeichnet, dass** die Dehydrierung in Gegenwart von Sauerstoff durchgeführt wird.

11. Verfahren nach einem der Ansprüche 1 bis 10, durch **gekennzeichnet**, dass die Dehydrierung in Gegenwart von Sauerstoff und Wasserdampf durchgeführt wird.

## Claims

1. A method for dehydrogenating primary or secondary alcohols having 1 to 12 carbon atoms to give the corresponding aldehydes or ketones in which the alcohol is brought into contact with a catalytically active composition comprising an active component of the formula
PdₐBi_{b}Y_{c}Z_{d},
where
Y is selected from the group consisting of Co, Rh, Pt, Ag, Au, and
Z is selected from the group consisting of Na, Cs, Mg, Ca, Ba, V, Cr, W, Fe, Ni, Cu, Sb
where the indices a, b, c and d give the mass ratios of the respective elements to one another, where
a = 0.1 - 3,
b = 0.1 - 3,
c=0-3,
d = 0 - 1.

2. The method according to claim 1, wherein the active component has the formula
PdₐBi_{b}Y_{c},
with Y = Au or Rh, where
a = 0.1 - 3,
b = 0.1 - 3,
c=0-3.

3. The method according to claim 1 or 2, c being at least 0.1.

4. The method according to claim 1, wherein the active component has the formula
PdₐBi_{b}Rh_{c},
where
a = 0.1 - 3,
b = 0.1 - 3,
c = 0.1 - 3.

5. The method according to claim 1, wherein the active component has the formula
PdₐBi_{b}RhₑY_{f},
with Y = Ag or Pt, where
a = 0.1 - 3,
b = 0.1 - 3,
e = 0,1 - 3,
f = 0 - 1,
e + f ≤ 3.

6. The method according to claim 1, wherein the active component has the formula
PdₐBi_{b}Co_{c},
where
a = 0.1 - 3,
b = 0.1 - 3,
c = 0.1 - 1.

7. The method according to claim 1, wherein the active component has one of the formulae below
- **Pd**_{0.5-1.0}**Rh**_{0.5-1.25}**Bi**_{1.25-175}**Ag**_{0.05-0.15}
- **Pd**_{0.5-1.0}**Rh**_{1.0-1.5}**Bi**_{0.75-1.25}**Pt**_{0.01-0.1}
- **Pd**_{0.25-0.5}**Rh**_{1.75-2.5}**Bi**_{025-0.5}**Co**_{0.01-0.1}
- **Pd**_{0.5-1.25}**Rh**_{0.5-1.25}**Bi**_{0.75-1.5}**Cr**_{0.01-0.1}
- **Pd**_{1.0-1.75}**Rh**_{0.25-0.75}**Bi**_{0.75-1.5}**Pt**_{0.0-0.15}**Co**_{0.01-0.1}
- **Pd**_{1.0-1.75}**Rh**_{0.25-0.75}**Bt**_{0.75-1.5}**Pt**_{0.05-0.15}
- **Pd**_{0.5-1.0}**Rh**_{1.0-1.75}**Bi**_{0.5-1.25}**Ag**_{0.03-0.15}**Ca**_{0.02-0.1}
- **Pd**_{0.4-1.0}**Rh**_{1.0-1.75}**Bi**_{0.75-1.25}**Ag**_{0.03-0.15}
- **Pd**_{1.25-1.75}**Bi**_{1.25-1.75}**Co**_{0.005-0.02}
- **Pd**_{0.4-1.0}**Rh**_{1.0-1.75}**Bi**_{0.75-1.25}
- **Pd**_{0.15-2.25}**Rh**_{0-2.5}**Bi**_{0.15-2.75},

8. The method according to one of claims 1 to 7, wherein the active component is present on a support material selected from the group consisting of silicon carbides, silicon nitrides, carbon nitrides, oxonitrides, oxocarbides, bismuth oxide, titanium oxide, zirconium oxide, boron nitride, aluminum oxide, silicates, aluminosilicates, zeolitic and also zeolite-analogous materials, steatite, activated carbon, metal meshes, stainless steel meshes, steel meshes and also mixtures of two or more of the abovementioned support materials.

9. The method according to one of claims 1 to 8, wherein the alcohol to be dehydrogenated is selected from methanol, ethanol, n-propanol and isopropanol.

10. The method according to one of claims 1 to 9, wherein the dehydrogenation is carried out in the presence of oxygen.

11. The method according to one of claims 1 to 10, wherein the dehydrogenation is carried out in the presence of oxygen and steam.

## Revendications

1. Procédé pour la déshydrogénation d'alcools primaires ou secondaires ayant de 1 à 12 atomes de carbone en les aldéhydes correspondants ou les cétones correspondantes, dans lequel on met l'alcool en contact avec une composition catalytiquement active comprenant un composant actif de formule
PdₐBi_{b}Y_{c}Z_{d}
dans laquelle
Y est choisi dans le groupe constitué par CO, Rh, Pt, Ag, Au, et
Z est choisi dans le groupe constitué par Na, Cs, Mg, Ca, Ba, V, Cr, W, Fe, Ni, Cu, Sb
et dans laquelle les indices a, b, c et d indiquent les rapports massiques des éléments respectifs entre eux, où
a = 0,1 - 3,
b = 0,1 - 3,
c = 0 - 3,
d = 0 - 1.

2. Procédé selon la revendication 1, **caractérisé en ce que** le composant actif présente la formule
PdₐBi_{b}Y_{c}
où Y = Au ou Rh et où
a = 0,1 - 3,
b = 0,1 - 3,
c = 0 - 3.

3. Procédé selon la revendication 1 ou 2, dans lequel c est au moins égal à 0,1.

4. Procédé selon la revendication 1, **caractérisé en ce que** le composant actif présente la formule
PdₐBi_{b}Rh_{c}
où
a = 0,1 - 3,
b = 0,1 - 3,
c = 0,1 - 3.

5. Procédé selon la revendication 1, **caractérisé en ce que** le composant actif présente la formule
PdₐBi_{b}RhₑY_{f}
où Y = Ag ou Pt, et où
a = 0,1 - 3,
b = 0,1 - 3,
e = 0,1 - 3.
f = 0 - 1,
e + f ≤ 3.

6. Procédé selon la revendication 1, **caractérisé en ce que** le composant actif présente la formule
PdₐBi_{b}CO_{c}
où
a = 0,1 - 3,
b = 0,1 - 3,
c = 0,1 - 1.

7. Procédé selon la revendication 1, **caractérisé en ce que** le composant actif présente l'une des formules suivantes
- Pd_{0,5-1,0}Rh_{0,5-1,25}Bi_{1,25-1,75}Ag_{0,05-0,15}
- Pd_{0,5-1,0}Rh_{1,0-1,5}Bi_{0,75-1,25}Pt_{0,01-0,1}
- Pd_{0,25-0,5}Rg_{1,75-2,5}Bi_{0,25-0,5}Co_{0,01-0,1}
- Pd_{0,5-1,25}Rh_{0,5-1},₂₅Bi_{0,75-1,5}Cr_{0,01-0,1}
- Pd_{1,0-1,75}Rh_{0,25-0,75}Bi_{0,75-1,5}Pt_{0,0-0,15}Co_{0,01-0,1}
- Pd_{1,0-1,75}Rh_{0,25-0,75}Bi_{0,75-1,5}Pt_{0,05-0,115}
- Pd_{0,5-1,0}Rh_{1,0-1,75}Bi_{0,5-1,25}Ag_{0,03-0,15}Ca_{0,02-0,1}
- Pd_{0,4-1,0}Rh_{1,0-1,75}Bi_{0,75-1,25}Ag_{0,03-0,15}
- Pd_{1,25-1,75}Bi_{1,25-1,75}Co_{0,005-0,02}
- Pd_{0,4-1,0}Rh_{1,0-1,75}Bi_{0,75-1,25}
- Pd_{0,15-2,25}Rh_{0-2,5}Bi_{0,15-2,75}

8. Procédé selon l'une quelconque des revendications 1 à 7, **caractérisé en ce que** le composant actif se trouve sur une matière de support choisie dans le groupe constitué par les carbures de silicium, les nitrures de silicium, les carbonitrures, les oxonitrures, les oxocarbures, l'oxyde de bismuth, l'oxyde de titane, l'oxyde de zirconium, le nitrure de bore, l'oxyde d'aluminium, les silicates, les aluminosilicates, des matières zéolithiques ainsi que des matières analogues aux zéolithes, la stéatite, le charbon actif, des toiles métalliques, des toiles en acier spécial, des toiles en acier ainsi que des mélanges de deux ou plus de deux des matières de support précitées.

9. Procédé selon l'une quelconque des revendications 1 à 8, **caractérisé en ce que** l'alcool à déshydrogéner est choisi parmi le méthanol, l'éthanol, le n-propanol et l'isopropanol.

10. Procédé selon l'une quelconque des revendications 1 à 9, **caractérisé en ce que** la déshydrogénation est effectuée en présence d'oxygène.

11. Procédé selon l'une quelconque des revendications 1 à 10, **caractérisé en ce que** la déshydrogénation est effectuée en présence d'oxygène et de vapeur d'eau.
